# EUROPÄISCHE PATENTSCHRIFT

(11) **EP 1 815 760 B1**
(45) Veröffentlichungstag und Bekanntmachung des Hinweises auf die Patenterteilung: **01.09.2010**
(21) Anmeldenummer: 07000586.3
(22) Anmeldetag: 12.01.2007
(51) Int. Cl.: A41D 19/00, A61B 5/024

(54) **Trägereinheit zum Halten einer Messeinheit an einer Hand**
Carrier for attaching a measuring unit to one hand
Support pour attacher une unité de mesure à une main

(30) Priorität: 06.02.2006 DE 102006005211
(43) Veröffentlichungstag der Anmeldung: 08.08.2007
(73) Patentinhaber: Beurer GmbH, 89077 Ulm (DE)
(72) Erfinder: Roschk, Patricio, 87435 Kempten (DE); Bühler, Marco, 89077 Ulm (DE); Merk, Ernst, 89264 Weissenhorn (DE); Meternek, Werner, 89233 Neu-Ulm (DE)
(74) Vertreter: Fleck, Hermann-Josef

(56) Entgegenhaltungen:
- DE-U1-202006 009 103
- GB-A- 1 165 103
- US-A- 4 281 389
- US-A- 5 749 841

## Beschreibung

Die Erfindung bezieht sich auf eine Trägereinheit zum Halten einer Messeinheit an einer Hand mit einem Grundträger, der als Handschuhhalter mit einer Fingerschlaufe ausgebildet ist und auf dessen Außenseite über dem Handrücken ein Koppelabschnitt angeordnet ist, auf dem ein zusätzlich vorhandener Messeinheitträger angebracht oder anbringbar ist, welcher mit Haltemitteln zum Aufbringen und Fixieren eines einen Anzeigeteil aufweisenden Auswerteabschnitts der Messeinheit versehen ist.

Eine derartige Trägereinheit zum Halten einer Messeinheit an einer Hand ist in der US-A 4,281,389 angegeben. Bei dieser bekannten Trägereinheit wird eine Messeinheit in Form einer Uhr für die Zwecke eines Läufers an einem Grundträger gehalten, der als eine Art Handschuh ausgebildet ist. Dieser Handschuhträger, der eine Fingerschlaufe für einen Zeigefinger und ein Daumenloch aufweist, ist im Bereich des Handrückens mit einem Koppelabschnitt versehen, an dem mittels eines Bandes die Messeinheit mit ihrem einen Anzeigeteil aufweisenden Auswerteabschnitt anbringbar ist. Mit der Messeinheit und dem Auswerteabschnitt sind verschiedene Messwerte erfassbar, wie z. B. Laufgeschwindigkeit und Pulsrate, und über den Anzeigeteil anzeigbar.

Die GB-A 1 165 103 zeigt eine weitere Trägereinheit in Form eines Handschuhhalters mit einem im Bereich des Handrückens angeordneten Koppelabschnitts, auf den eine Zeitmesseinheit mittels eines Haltestreifens durch Klettverbindung fixierbar ist. Der Handschuhträger weist für die einzelnen Finger jeweils Fingerlöcher und ein Daumenloch auf.

Die US-A 5,749,841 zeigt eine weitere Trägereinheit zum Halten einer Messeinheit, die als Handschuhhalter ausgebildet ist, der eine große Öffnung gemeinsam für alle Finger und ein separates Daumenloch aufweist. Im Bereich des Handgelenks ist auf der Gelenkrückseite ein Auswerteabschnitt der Messeinheit mit Anzeigeteil mittels eines Handgelenkbandes und eines daran fixierten weiteren Bandes angebracht. Auch hierbei dient zur Fixierung eine Klettverbindung.

Eine weitere Trägereinheit ist, wie die EP 1 468 645 A9 zeigt, üblicherweise als Armband ausgebildet, auf dessen Außenseite eine Messeinheit für physiologische Signale, im dargestellten Fall Herzpulse, aufgenommen ist. Die Messeinheit erhält dabei ihre Signale von einem in einem Brustgurt angeordneten Messaufnehmer mit Sensor über eine kurze Funkstrecke. Über die Messeinheit mit einem integrierten Bedienungs- und Anzeigeteil können die den Benutzer interessierenden Werte leicht abgelesen werden. Ähnliche Trägereinheiten in Verbindung mit einer Fitness-Messeinheit zeigen auch die DE 299 10 633 U1 und die DE 696 23 314 T2.

Derartige Messaufnehmer stellen jedoch nicht für jeden Benutzer die optimale Lösung dar und es gibt auch andere Möglichkeiten, physiologische Messwerte für einen Trainierenden zu erfassen, wobei ebenfalls ein Anzeigeteil im Handbereich zweckmäßig ist.

Der Erfindung liegt die Aufgabe zugrunde, eine Trägereinheit der eingangs genannten Art bereit zu stellen, mit der die Möglichkeiten der Anbringung der Messeinheit gegebenenfalls in Verbindung mit anderen Messaufnehmern verbessert werden.

Diese Aufgabe wird mit den Merkmalen des Anspruches 1 gelöst. Hierbei ist vorgesehen, dass die Fingerschlaufe mit öffenbaren und in verschiedenen Weiten verschließbaren Verschlussmitteln versehen ist.

Der Aufbau der Trägereinheit mit der Anordnung des Auswerteabschnitts mit der Anzeige im Bereich des Handrückens ergibt zum einen eine günstige Ablesestellung für den Trainierenden in günstigem Betrachtungswinkel. Zudem bietet die Trägereinheit erweiterte Möglichkeiten für die Positionierung und damit Ausbildung eines Messwertaufnehmers, beispielsweise zur Herzpulsfrequenzmessung, wie etwa einen Sensor bei einem plethysmographisch arbeitenden Gerät. Beispielsweise können mittels des Handschuhhalters vorteilhaft optische Sensoren, ähnlich wie sie von Aufklemmgebern her bekannt sind, fixiert und in Position gehalten werden. Mittels des Messeinheitträgers wird der Auswerteabschnitt mit dem Anzeigeteil, die vorteilhafterweise in einem gemeinsamen Gehäuse zusammen mit Bedienungselementen und Verarbeitungskomponenten für die Messsignale untergebracht sind, sicher gehalten werden. In der Fingerschlaufe kann günstig ein Sensor untergebracht werden, wobei vorgesehen ist, dass die Fingerschlaufe mit öffenbaren und in verschiedenen Weiten verschließbaren Verschlussmitteln versehen ist.

Dabei besteht eine vorteilhafte Ausgestaltung darin, dass der Messeinheitträger mindestens eine zusätzliche Schicht auf der Außenseite des Handschuhhalters bildet. Durch die zusätzliche Schicht auf der Außenseite des Handschuhhalters kann einerseits der Handschuhhalter an die Erfordernisse zum Aufrechterhalten einer geeigneten Temperatur an der Hand und zum Abführen von Feuchtigkeit ausgelegt werden, während andererseits der Messeinheitträger zum sicheren Halten und zum Schutz der Messeinheit ausgebildet werden kann.

Ist vorgesehen, dass der Koppelabschnitt mit Klettverbindungsmitteln versehen ist und dass der Messeinheitträger auf seiner im angebrachten Zustand dem Handrücken zugekehrten Unterseite mit weiteren Klettverbindungsmitteln versehen und mittels dieser auf dem Koppelabschnitt lösbar fixierbar ist, so wird nicht nur eine einfache Handhabung beim Anbringen und Abnehmen der Messeinheit ermöglicht, sondern die beiderseitigen Klettverbindungsmittel ergeben auch eine Durchlüftungsschicht zum Abtransport von Feuchtigkeit.

Eine sichere Anbringung des Messeinheitträgers und damit auch der Messeinheit wird dadurch unterstützt, dass der Messeinheitträger - gegebenenfalls zusätzlich - mit einer öffenbaren Handgelenkschlaufe am Handgelenk anbringbar ist.

Die Anbringung der Messeinheit kann einfach dadurch vorgenommen werden, dass der Messeinheitträger auf seiner Oberseite mit Haltemitteln zum Anbringen des Auswerteabschnittes versehen ist.

Mit den Maßnahmen, dass der Handschuhhalter aus mindestens einem feuchtigkeitsdurchlässigen Material hergestellt ist und dass der Messeinheitträger ein feuchtigkeitsaufnehmendes Material aufweist, wird einerseits die Feuchtigkeit von der Hand eines Trainierenden effektiv abgeführt und andererseits ein Schutz der Messeinheit gegen Feuchtigkeit erreicht.

Für die Handhabung und Haltefunktion sind des Weiteren die Maßnahmen von Vorteil, dass der Handschuhhalter eine öffenbare Handgelenkschlaufe sowie ein Daumenloch aufweist. Für eine eindeutige Positionierung und sichere Fixierung eines Sensors sind weiterhin die Maßnahmen von Vorteil, dass der Handschuhhalter mit einem Sensorhalter für einen Sensor der Messeinheit versehen ist. Beispielsweise kann der Sensorhalter eine Tasche, ein Klemmmittel, z.B. ein Druckknopf, oder einfach eine knopflochartige Öffnung in dem Handschuhhalter sein.

Damit ein Kabel der Messeinheit, beispielsweise ein Verbindungskabel zwischen Auswerteabschnitt und Sensor, sich nicht störend auswirkt, ist vorgesehen, dass der Handschuhhalter mit einem Kabelhalter für ein Kabel der Messeinheit versehen ist. Alternativ kann eine Verbindungsleitung in den Handschuhhalter integriert sein.

Die Erfindung wird nachfolgend anhand von Ausführungsbeispielen unter Bezugnahme auf die Zeichnungen näher erläutert. Es zeigen:
- Fig. 1: eine Zusammenstellung wesentlicher Teile einer Trägereinheit mit Messeinheit im auseinander genommenen Zustand in perspektivischer Darstellung,
- Fig. 2: die Trägereinheit nach Fig. 1 mit der Messeinheit im zusammengesetz- ten Zustand,
- Fig. 3: die Trägereinheit mit aufgenommener Messeinheit in einem an der Hand angelegten Zustand,
- Fig. 4: eine weitere Darstellung der Trägereinheit mit Messeinheit in an der Hand angelegtem Zustand in anderer Perspektive,

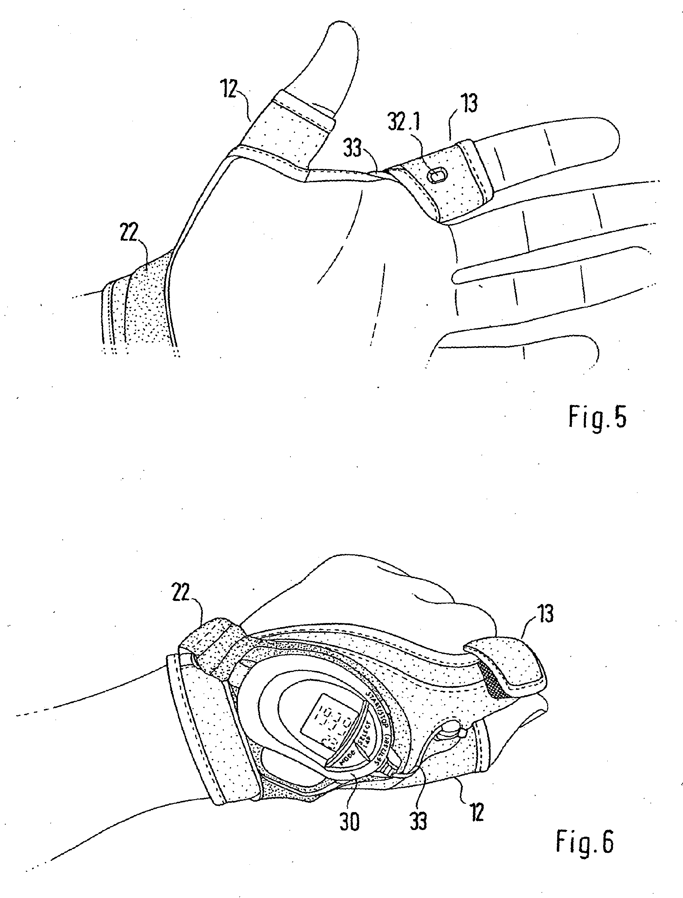

- Fig. 5: eine weitere Darstellung der an der Hand angelegten Trägereinheit mit Messeinheit,
- Fig. 6: eine weitere Darstellung der an der Hand angelegten Trägereinheit mit Messeinheit,
- Fig. 7: eine weitere Darstellung der an der Hand angelegten Trägereinheit mit Messeinheit und
- Fig. 8: eine teilweise aufgeschnittene Darstellung der an der Hand angelegten Trägereinheit mit Messeinheit in seitlicher Ansicht.

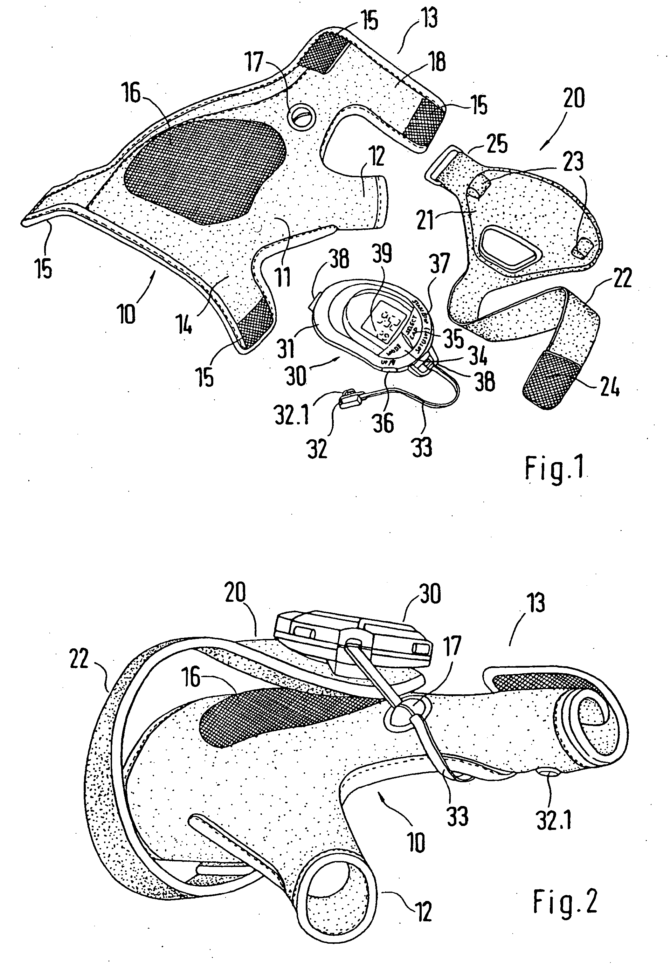

Fig. 1 zeigt als wesentliche Teile einer Trägereinheit einen Handschuhhalter 10 und einen auf dessen Handrückenteil 11 an einem dort befindlichen Koppelabschnitt 16 fixierbaren Messeinheitträger 20 zum Aufnehmen einer Messeinheit 30, die vorliegend von einer Pulsuhr gebildet wird.

Die Messeinheit 30 weist ein auf dem Messeinheitträger 20 ankoppelbares Gehäuse mit einem Anzeigeteil 39, verschiedenen Bedientasten, wie Betriebsarttaste 34, Funktionstaste 35, weitere Taste 36, Start/Stopptaste 37 und beiderseitigen Verbindungsabschnitten 38 sowie einen an das Gehäuse über ein Kabel 33 angeschlossenen Sensor 32 in Form eines optischen Fingersensors auf. Das Gehäuse umfasst einen Auswerteabschnitt 31 mit einer in dem Gehäuse untergebrachten Verarbeitungseinrichtung für die von dem Sensor 32 über das Kabel 33 oder alternativ kabellos gelieferten Signale, insbesondere Herzpulsfrequenzsignale, die mittels an sich bekannter Hardware und/oder Software ausgewertet werden. Verschiedene Sensortypen sind denkbar, beispielsweise solche für eine plethysmographische Messung. Auf seiner von der Fingerfläche abgewandten Oberseite weist der Sensor 32 einen knopfartigen Halteabschnitt 32.1 auf, mit dem der Sensor 32 an einem Sensorhalter 18 mit knopflochartiger Öffnung an dem Handschuhhalter 10 anbringbar ist.

Der Handschuhhalter 10 besitzt ein stutzenförmiges Daumenloch 12, eine öffenbare Fingerschlaufe 13, die insbesondere an dem Zeigefinger der betreffenden, vorzugsweise linken Hand anlegbar und verschließbar ist, sowie eine um das Handgelenk legbare und dort verschließbare Handgelenkschlaufe 14. Sowohl die Fingerschlaufe 13 als auch die Handgelenkschlaufe 14 tragen als öffenbare Verschlussmittel jeweilige Klettverschlussabschnitte 15, so dass sie weitgehend beliebig an Handgelenke bzw. Finger verschiedener Umfangsweiten angepasst werden können. Die Öffnung des Sensorhalters 18 ist in der Fingerschlaufe 13 an einer Stelle positioniert, die im Wesentlichen mit der Unterseite des betreffenden Fingers zur Deckung kommt, so dass der fixierte Sensor 32 in diesem Bereich positioniert wird.

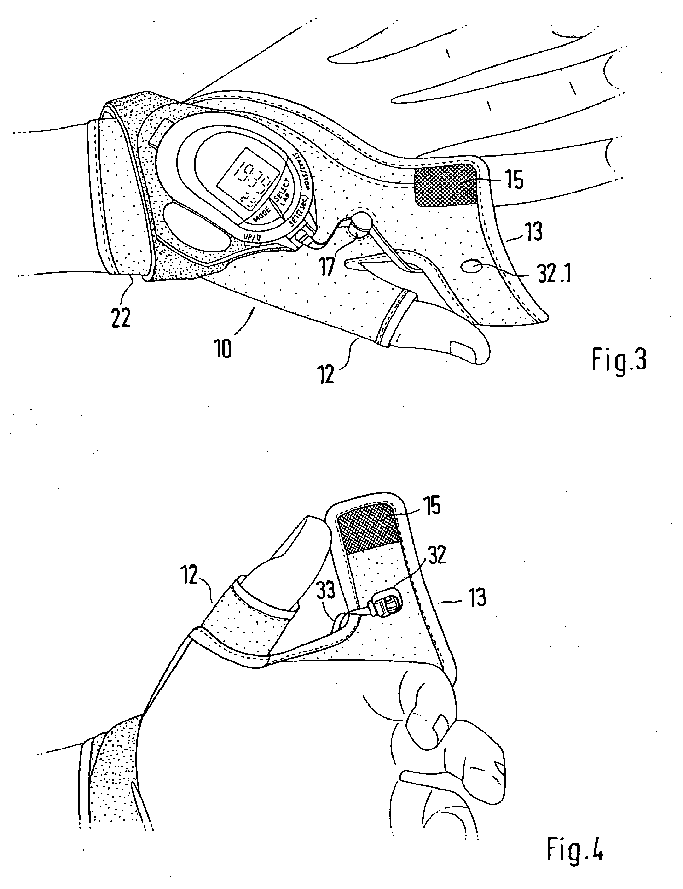

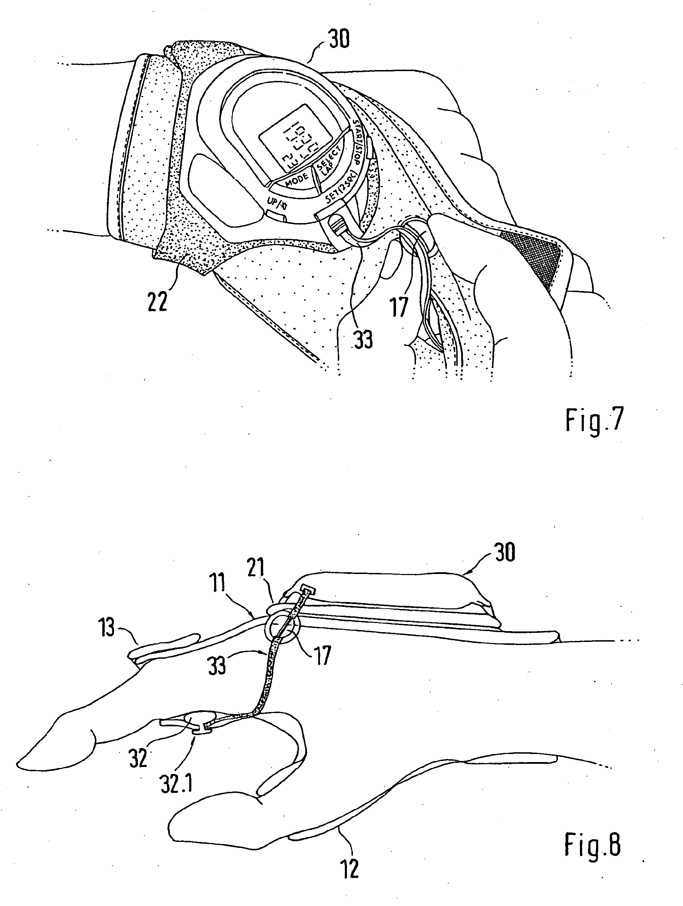

Der auf dem Handrückenteil 11 angeordnete Koppelabschnitt 16 des Handschuhhalters 10 ist ebenfalls als Klettverbindung ausgebildet, um darauf den Messeinheitträger 20 festzulegen, der an seiner dem Handrücken zugekehrten Unterseite ein entsprechendes Gegenelement in Form einer Klettverbindung trägt. Zwischen dem Koppelabschnitt 16 und dem Sensorhalter 18 ist der Handschuhhalter 10 auf seiner von der Handfläche abgewandten Oberseite mit einem Kabelhalter 17 ausgestattet, der einen elastisch öffenbaren hinterschnittenen Halteschlitz für das Kabel 33 aufweist, wie z.B. auch die Fig. 2, 3 und 7 zeigen. Das Kabel 33 kann in den elastisch aufweitbaren Schlitz eingeführt und, wie aus Fig. 7 ersichtlich, durch beiderseitiges Drücken des Kabelhalters 17 geöffnet werden, um das Kabel herauszunehmen.

Alternativ zu dem freien Kabel 33 kann in den Handschuhhalter 10 eine Verbindungsleitung integriert sein, beispielsweise als flexible Leiterplatte, als Schicht, als Einwebung in das Textil oder als leitender Textilabschnitt, insbesondere Faden.

Der Messeinheitträger 20 weist auf seiner Oberseite eine Schicht aus Naturleder auf und ist seitlich mit Haltemitteln 23 versehen, die als öffenbare Klettschlaufen 23 ausgebildet sind und durch die ösenartigen Verbindungsabschnitte 38 der Messeinheit 30 hindurchführbar sind, um die Messeinheit 30 auf der Oberseite des Messeinheitträgers 20 positionsgenau und sicher zu halten. Der Zuschnitt des Messeinheitträgers 20 weist drei zungenartige Ausformungen auf. An einer zungenartigen Ausformung befindet sich eine Verbindungsöse 25, während an einer weiteren zungenartigen Ausformung eine Handgelenkschlaufe 22 angebunden ist, die an ihrem Endabschnitt mit einem Verschlussmittel 24, insbesondere Klettverbindern versehen ist, um die um das Handgelenk gelegte Handgelenkschlaufe 22 zu verschließen. Der aus Leder ausgebildete Aufnahmeabschnitt 21 besitzt eine gute Wasserspeicherfähigkeit und kann daher evtl. von dem Handrückenteil 11 eindringende Feuchtigkeit aufnehmen, so dass die Messeinheit 30 auf ihrer Unterseite vor Feuchtigkeit geschützt wird.

Auch die Materialauswahl des Handschuhhalters 10 trägt zum Abtransport von Feuchtigkeit an der Hautoberfläche bei. In dem Bereich des Koppelabschnittes 16 auftretende Feuchtigkeit wird zudem durch das dort in Folge der Klettverschlusselemente gebildete Luftpolster abgeführt. Durch den Aufbau des Handschuhhalters 10 wird einerseits die Hand angenehm temperiert und andererseits Feuchtigkeit von der Messeinheit 30 ferngehalten. Als Material für den Handschuhhalter 10 eignet sich z.B. Neopren. Dabei kommt z.B. eine Zusammensetzung von ca. 30 % Chloropren-Kautschuk und ca. 70 % Styrol-Butadien-Kautschuk in Betracht, wobei jeweilige Abweichungen von 10 % oder 20 % nach oben oder unten auch noch in Frage kommen können. Die Borde können aus hoch elastischem Polyurethan hergestellt werden.

Der Kabelhalter 17 besteht vorzugsweise aus elastischem Kunststoff. Mit dem Handschuh wird auch ein guter thermischer Schutz der Hand insbesondere auch im Bereich des mit dem Sensor 32 in Verbindung gebrachten Fingers erreicht, so dass eine gleichmäßige Durchblutung und zuverlässige Messergebnisse bei der z.B. plethysmographischen Messung unterstützt werden.

Für den Messeinheitträger 20 kommen für das Trägermaterial mit der bandartigen Handgelenkschlaufe auch z.B. Chloropren-Kautschuk und Styrol-Butadien-Kautschuk der vorstehend genannten Zusammensetzung in Betracht, während als Obermaterial Leder wegen des genannten Vorteils der Wasserspeicherfähigkeit günstig ist.

Um auch im Winter bei niedrigen Temperaturen für eine gute Durchblutung der Hand zu sorgen, kann die Trägereinheit als Winter-Trägereinheit zusätzlich mit einem an den Handschuhhalter 10 mit dem Messeinheitträger 20 und der Messeinheit 30 angepassten Winterhandschuh ausgestaltet sein. Während der Handschuh für die andere Hand ein normaler Winterhandschuh sein kann, ist der über den Handschuhhalter 10 ziehbare Winterhandschuh, in der Regel der linken Hand, an seiner Oberseite mit einer Öffnung für die Messeinheit versehen. Dies ermöglicht auch beim Winterbetrieb eine Überwachung des Trainings, wobei auch ein Betrieb der Messeinheit 30, beispielsweise also der Pulsuhr, auch bei sehr niedrigen Temperaturen möglich ist. Eine weitere Ausgestaltung besteht dabei darin, dass das Gehäuse der Messeinheit 30 mit dem Auswerteabschnitt 31 mit einem transparenten Kunststoff abgedeckt ist, so dass auch ein Schutz vor Feuchtigkeit von außen gegeben ist. Als Materialien für den Winterhandschuh ist z.B. Lycra (Polyamid-ummanteltes Elastan) geeignet, während für die Borde hochelastisches Polyurethan und für den Verschluss am Handgelenk ebenfalls eine Klettverbindung vorteilhaft sind. Grundsätzlich sind als funktionelle Textilien atmungsaktive und (thermisch) isolierende Textilien geeignet, die im Schichtaufbau einlagig oder mehrlagig verwendet werden können. Von der Materialbeschaffenheit her kommen Mikrofaser bzw. Mikro-Hohlfaser in Betracht. Vom Ursprung her können die Materialien aus künstlich hergestellten Rohstoffen oder natürlichen Rohstoffen gewonnen werden. Auch können die Materialien wasser- und schmutzabweisend beschichtet sein, beispielsweise nach Art des Lotusblüteneffekts, der Haifischhaut oder dgl. Auch Gesichtspunkte der Haptik und Griffigkeit sind bei den Materialien berücksichtigt, wobei eine Verbesserung der Griffigkeit an den Fingern und der Unterhand durch Verwendung verschiedener Materialien und/oder der Oberflächenbeschichtung und Beschaffenheit gewährleistet wird. Dabei sind auch Eigenschaften hinsichtlich Rutschfestigkeit und Optik beachtet. Für eine gute Anpassbarkeit an verschiedene Formen eignen sich elastische Materialien. Neben Klettverbindern als Verschlussmittel kommen beispielsweise auch Schnappverschlüsse oder Schnürungen in Betracht. Derartige Ausgestaltungen in der Materialbeschaffenheit und dem Aufbau sind auch, soweit das jeweils zweckmäßig ist, für den Handschuhhalter 10 einsetzbar.

Weitere Ausgestaltungen bestehen darin, dass an dem Handschuhhalter 10 oder den Winterhandschuh Reflektoren angebracht sind, um den Trainierenden auch bei schlechter Sicht gut zu erkennen.

Der Winterhandschuh kann auch eine Beheizung für die Aufrechterhaltung der Temperatur und Durchblutungsförderung der Haut aufweisen.

Ferner kann der Handschuhhalter 10 und/oder der Winterhandschuh selbst mit einer integrierten Bedienung durch z.B. Druckknöpfe am Zeigefinger, welche mit dem Daumen betätigt werden, oder durch Druck-Reaktionstextilien, wie Folienknöpfe/-Tastatur oder Druckschalter versehen sein. In dem Handschuhhalter 10 bzw. dem Winterhandschuh können zudem Leuchtfunktionen integriert sein, beispielsweise für eine Beleuchtung, für eine Hinweisfunktion oder für eine Warnfunktion, wobei als Lichtquellen Leuchtdioden herkömmlichen Aufbaus, organische Leuchtdioden oder EL-Folien in Betracht kommen. Die Beleuchtung kann dabei intermittierend (blinkend oder blitzend), stetig oder in einem Warnblinkmodus ausgeführt sein. Verschiedene Farben kommen in Betracht. Die Energieversorgung kann dabei über die Messeinheit oder eine eigenständige Batterie oder einen Solarbetrieb erfolgen.

Die beschriebene Trägereinheit stellt eine kompakte, einfach handhabbare Einheit mit vielfältigen Funktionsmöglichkeiten dar.

## Patentansprüche

1. Trägereinheit zum Halten einer Messeinheit (30) an einer Hand mit einem Grundträger, der als Handschuhhalter (10) mit einer öffenbaren Handgelenkschlaufe (14), einer Fingerschlaufe (13) sowie einem Daumenloch (12) ausgebildet ist und auf dessen Außenseite über dem Handrücken ein Koppelabschnitt (16) angeordnet ist, auf dem ein zusätzlich vorhandener Messeinheitträger (20) angebracht oder anbringbar ist, welcher mit Haltemitteln (23) zum Aufbringen und Fixieren eines einen Anzeigeteil (39) aufweisenden Auswerteabschnitts (31) der Messeinheit (30) versehen ist,
**dadurch gekennzeichnet,**
**dass** die Fingerschlaufe (13) mit öffenbaren und in verschiedenen Weiten verschließbaren Verschlussmitteln versehen ist.

2. Trägereinheit nach Anspruch 1,
**dadurch gekennzeichnet,**
**dass** der Messeinheitträger (20) mindestens eine zusätzliche Schicht auf der Außenseite des Handschuhhalters (10) bildet.

3. Trägereinheit nach Anspruch 1 oder 2,
**dadurch gekennzeichnet,**
**dass** der Koppelabschnitt (16) mit Klettverbindungsmitteln versehen ist und dass der Messeinheitträger (20) auf seiner im angebrachten Zustand dem Handrücken zugekehrten Unterseite mit weiteren Klettverbindungsmitteln versehen und mittels dieser auf dem Koppelabschnitt (16) lösbar fixierbar ist.

4. Trägereinheit nach einem der vorhergehenden Ansprüche,
**dadurch gekennzeichnet,**
**dass** der Messeinheitträger (20) - gegebenenfalls zusätzlich - mit einer öffenbaren Handgelenkschlaufe (22) am Handgelenk anbringbar ist.

5. Trägereinheit nach einem der vorhergehenden Ansprüche,
**dadurch gekennzeichnet,**
**dass** der Messeinheitträger (20) auf seiner Oberseite mit den Haltemitteln (23) zum Anbringen des Auswerteabschnittes (31) versehen ist.

6. Trägereinheit nach einem der vorhergehenden Ansprüche,
**dadurch gekennzeichnet,**
**dass** der Handschuhhalter (10) aus mindestens einem feuchtigkeitsdurchlässigen Material hergestellt ist und
**dass** der Messeinheitträger (20) ein feuchtigkeitsaufnehmendes Material aufweist.

7. Trägereinheit nach einem der vorhergehenden Ansprüche,
**dadurch gekennzeichnet,**
**dass** der Handschuhhalter (10) mit einem Sensorhalter (18) für einen Sensor (32) der Messeinheit (30) versehen ist.

8. Trägereinheit nach Anspruch 7,
**dadurch gekennzeichnet,**
**dass** der Sensorhalter (18) in der Fingerschlaufe (13) positioniert ist.

9. Trägereinheit nach einem der vorhergehenden Ansprüche,
**dadurch gekennzeichnet,**
**dass** der Handschuhhalter (10) mit einem Kabelhalter (17) für ein Kabel (33) der Messeinheit (30) versehen ist oder
**dass** eine elektrische Verbindungsleitung zwischen dem Auswerteabschnitt (31) und dem Sensor (32) in den Handschuhhalter (10) integriert ist.

## Claims

1. Carrier unit for holding a measuring unit (30) on one hand with a base carrier which is designed as a glove-type holder (10) with an opening wrist loop (14), a finger loop (13) and a thumb hole (12), and on the outside of which a coupling section (16) is arranged on the back of the hand, on which an additional measuring unit carrier (20) is or can be attached, which is provided with retaining means (23) for attaching and fixing a control section (31) of the measuring unit (30) having a display part (39),
**characterised in**
**that** the finger loop (13) is provided with opening means of closure which can be closed to various widths.

2. Carrier unit according to claim 1,
**characterised in**
**that** the measuring unit carrier (20) forms at least one additional layer on the outside of the glove holder (10).

3. Carrier unit according to claim 1 or 2,
**characterised in**
**that** the coupling section (16) is provided with hook-and-loop fastening means and that the measuring unit carrier (20) on its underside, facing the back of the hand in the attached condition, is provided with additional hook-and-loop fastening means and is detachably fixable by said means on the coupling section (16).

4. Carrier unit according to one of the preceding claims,
**characterised in**
**that** the measuring unit carrier (20) - additionally if necessary - can be attached to the wrist with an opening wrist loop (22).

5. Carrier unit according to one of the preceding claims,
**characterised in**
**that** the measuring unit carrier (20) is provided on its top side with said retaining means (23) to attach the control section (31).

6. Carrier unit according to one of the preceding claims,
**characterised in**
**that** the glove-type holder (10) is produced from at least one moisture-permeable material and
**that** the measuring unit carrier (20) has a moisture-absorbing material.

7. Carrier unit according to one of the preceding claims,
**characterised in**
**that** the glove-type holder (10) is provided with a sensor holder (18) for a sensor (32) of the measuring unit (30).

8. Carrier unit according to claim 7,
**characterised in**
**that** the sensor holder (18) is positioned in the finger loop (13).

9. Carrier unit according to one of the preceding claims,
**characterised in**
**that** the glove-type holder (10) is provided with a cable holder (17) for a cable (33) of the measuring unit (30) or
**that** an electrical connection lead between the control section (31) and the sensor (32) is integrated into the glove-type holder (10).

## Revendications

1. Unité de support conçue pour retenir une unité de mesure (30) sur une main, comportant un support de base réalisé sous la forme d'une mitaine de retenue (10) comprenant une boucle (14) d'articulation carpienne, apte à l'ouverture, une boucle (13) de logement d'un doigt, ainsi qu'un trou (12) de passage du pouce, et sur la face extérieure duquel se trouve, au-dessus du dos de la main, une zone de rattachement (16) sur laquelle est, ou peut être implanté un support additionnel (20) d'unité de mesure qui est muni de moyens de retenue (23) destinés à la mise en place, et à la consignation à demeure, d'une zone (31) de lecture interprétative de ladite unité de mesure (30), pourvue d'une partie d'affichage (39),
**caractérisée par le fait**
**que** la boucle (13) de logement d'un doigt est équipée de moyens de fermeture pouvant être ouverts, et fermés suivant différentes largeurs.

2. Unité de support selon la revendication 1,
**caractérisée par le fait**
**que** le support (20) de l'unité de mesure forme au moins une couche supplémentaire sur la face extérieure de la mitaine de retenue (10).

3. Unité de support selon la revendication 1 ou 2,
**caractérisée par le fait**
**que** la zone de rattachement (16) est équipée de moyens de liaison auto-agrippante ; et que le support (20) de l'unité de mesure offre, sur sa face inférieure tournée vers le dos de la main à l'état installé, d'autres moyens de liaison auto-agrippante à l'aide desquels il peut être verrouillé à demeure, de manière libérable, sur ladite zone de rattachement (16).

4. Unité de support selon l'une des revendications précédentes,
**caractérisée par le fait**
**que** le support (20) de l'unité de mesure peut - en plus, le cas échéant - être installé sur l'articulation carpienne à l'aide d'une boucle (22) d'articulation carpienne, apte à l'ouverture.

5. Unité de support selon l'une des revendications précédentes,
**caractérisée par le fait**
**que** le support (20) de l'unité de mesure est doté, sur sa face supérieure, des moyens de retenue (23) dévolus à la mise en place de la zone (31) de lecture interprétative.

6. Unité de support selon l'une des revendications précédentes,
**caractérisée par le fait**
**que** la mitaine de retenue (10) est fabriquée en au moins un matériau perméable à l'humidité ; et
**que** le support (20) de l'unité de mesure présente un matériau absorbant l'humidité.

7. Unité de support selon l'une des revendications précédentes,
**caractérisée par le fait**
**que** la mitaine de retenue (10) est munie d'un porte-capteur (18) destiné à un capteur (32) de l'unité de mesure (30).

8. Unité de support selon la revendication 7,
**caractérisée par le fait**
**que** le porte-capteur (18) se trouve dans la boucle (13) de logement d'un doigt.

9. Unité de support selon l'une des revendications précédentes,
**caractérisée par le fait**
**que** la mitaine de retenue (10) est dotée d'un porte-câble (17) dédié à un câble (33) de l'unité de mesure (30) ; ou
**qu'**une connexion par conducteur électrique est intégrée dans ladite mitaine de retenue (10), entre la zone (31) de lecture interprétative et le capteur (32).
